# DEMANDE DE BREVET EUROPEEN

(11) **EP 3 153 049 A1**
(43) Date de publication de la demande: **12.04.2017**
(21) Numéro de dépôt: 15306563.6
(22) Date de dépôt: 05.10.2015
(51) Int. Cl.: A43B 7/14, A43B 17/00, A43B 17/14, A61F 5/14

(54) **ARCHE DE VOÛTE PLANTAIRE POUR SEMELLE ORTHOPÉDIQUE ET SEMELLE ORTHOPÉDIQUE ASSOCIÉE**

(71) Demandeur: Otte, Christophe, 1205 Genève (CH)
(72) Inventeur: Otte, Christophe, 1205 Genève (CH)
(74) Mandataire: Brungard, Yves Francois

(57) **Abrégé**

L'arche de voute plantaire destinée à être montée sur une semelle orthopédique de rééducation, au droit de la voute plantaire d'un patient à traiter une coque de forme convexe élastiquement déformable.

Une semelle orthopédique de rééducation comportant une telle arche de voute plantaire, ainsi qu'un article de marche de pied comportant une telle semelle orthopédique sont décrits

## Description

L'invention concerne une arche de voute plantaire pour une semelle orthopédique de rééducation, ainsi qu'une semelle orthopédique de rééducation comportant une telle arche de voute plantaire.

Lors d'une journée, nous effectuons environs dix mille pas. Or pour 90% de la population, ces dix milles pas s'effectuent avec un mauvais positionnement. Ainsi, le fait de marcher et de se tenir debout sur des fondations en déséquilibre favorisent la survenue de tensions asymétriques au niveau des muscles du pied et occasionnent à la longue des troubles de la posture en chaine montante, troubles pouvant devenir invalidants.

Il est connu des semelles qui permettent d'améliorer la situation comme celles qui sont décrites dans le document EP 2 111 770. De telles semelles sont destinées à être insérés au sein d'une chaussure. Toutefois, ces semelles présentent l'inconvénient de n'être adaptées qu'à un instant particulier de la rééducation d'un patient. En effet, au cours de la rééducation aidée de telles semelles, le positionnement du pied du patient s'améliore et les semelles de l'art antérieur ne peuvent suivre cette évolution de manière optimale. Il est nécessaire de réaliser de nouvelles semelles orthopédiques de rééducation adaptées à cette évolution de la posture autant de fois que nécessaire. Ceci est complexe et lourd pour le patient.

Il y a donc nécessité de pouvoir fournir une semelle orthopédique de rééducation qui puisse évoluer de manière simple pour permettre une rééducation optimale du patient.

Un but de l'invention est de fournir une arche de voute plantaire à destination d'une semelle orthopédique de rééducation afin de la rendre simple et modulable en fonction du patient, tout en étant optimale dans la rééducation.

A cette fin, il est prévu, selon l'invention, une arche de voute plantaire comportant une coque de forme convexe élastiquement déformable et destinée à être montée de sur une semelle orthopédique de rééducation, au droit de la voute plantaire d'un patient à traiter.

Ainsi, le fait d'avoir une arche de voute plantaire comportant une coque convexe déformable élastiquement, montable sur une semelle orthopédique de rééducation permet d'obtenir une semelle orthopédique de rééducation qui soit simple à réaliser, facile d'utilisation, tout en étant modulable afin de suivre de manière optimale une rééducation d'un patient.

Avantageusement, mais facultativement, l'arche de voute plantaire présente au moins l'une des caractéristiques techniques suivantes additionnelles :
- la coque comporte une portion de cylindre de révolution ;
- la coque comporte une portion de coupelle, notamment une demi-coupelle ;
- la coque comporte un empilement de couches de matériaux souple ;
- la coque comporte une âme, une couche supérieure et une couche inférieure ;
- l'âme est réalisée en plastique injecté, notamment en thermoplastique ;
- l'âme est réalisée en un matériau à mémoire de forme ; et,
- la couche supérieure comporte un revêtement de fixation repositionnable, notamment un revêtement de type Velcro®.

Il est aussi prévu, selon l'invention, une semelle orthopédique de rééducation comportant une arche de voute plantaire qui présente au moins l'une des caractéristiques techniques précédentes.

Avantageusement, mais facultativement, la semelle orthopédique de rééducation présente au moins l'une des caractéristiques techniques additionnelles suivantes :
- la semelle comporte en outre une sangle de maintien de la semelle avec un pied d'un patient ; et,
- la sangle de maintien est amovible ;
- la semelle orthopédique comporte, sous elle, une semelle extérieure, l'arche de voute plantaire étant entre la semelle orthopédique et la semelle extérieure ; et,
- l'arche de voute plantaire et/ou la semelle extérieure et/ou la sangle de maintien est (sont) montée(s) de manière amovible(s).

Il est aussi prévu, selon l'intention, un article de marche de pied comportant une semelle orthopédique de rééducation présentant l'une des caractéristiques techniques précédentes.

D'autres avantages et caractéristiques de l'invention apparaîtront lors de la description ci après de deux modes de réalisations. Aux dessins annexés :
- la figure 1 est une vue en trois dimensions d'un premier mode de réalisation d'une arche de voute plantaire selon l'invention ;
- Les figures 2 et 3 sont des vues respectivement de coté et de dessus de l'arche de voute plantaire de la figure 1 ;
- la figure 4 est une vue en trois dimensions d'un deuxième mode de réalisation d'une arche de voute plantaire selon l'invention ;
- Les figures 5 et 6 sont des vues respectivement de dessus et de Coté de l'arche de voute plantaire de la figure 4 ;
- La figure 7a est une vue de dessous d'une semelle orthopédique de rééducation selon l'invention comportant une arche de voute plantaire selon la figure 4 ;
- La figure 7b est une vue de dessous d'une semelle orthopédique de rééducation selon l'invention comportant une arche de voute plantaire selon la figure 1 ;
- La figure 8 est une vue illustrant un article de marche de pied selon l'invention comportant une semelle orthopédique de rééducation selon l'invention des figures 7a et 7b ; et,
- Les figures 9a et 9b sont des vues schématiques de variantes de réalisation d'un article de marche de pied selon l'invention.

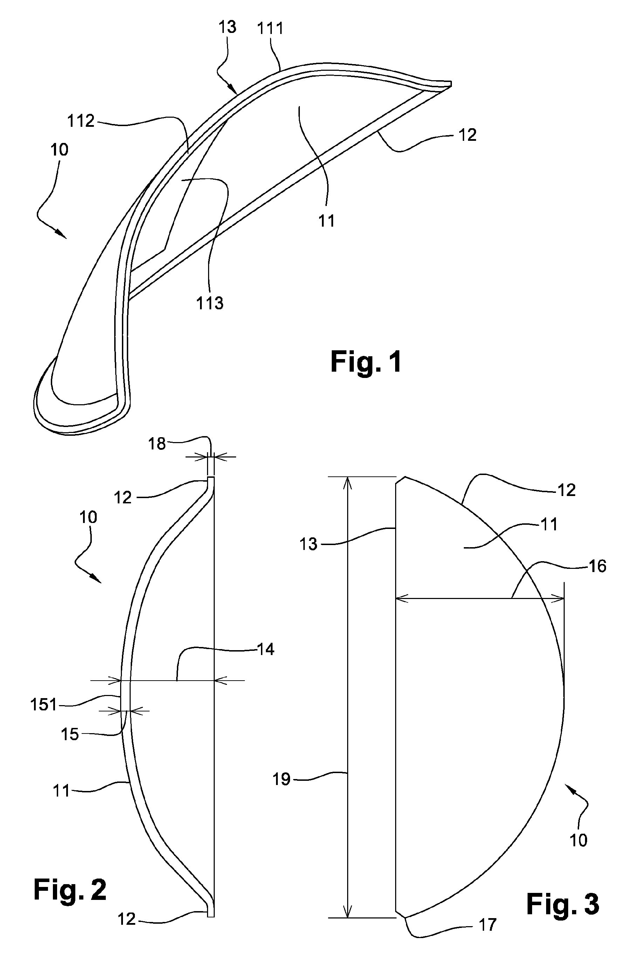

En référence aux figures 1 à 3, nous allons décrire en détail un premier mode de réalisation d'une arche de voute plantaire 10 selon l'invention.

L'arche de voute plantaire 10 selon l'invention comporte une coque de forme convexe 11. Dans le premier mode de réalisation de l'arche de voute plantaire 10 selon l'invention, la coque 11 comporte une portion de coupelle, ici une demi coupelle, comprenant un rebord 12 de forme de demi cercle sensiblement et un bord de tranche 13. Le rebord 12 et le bord de tranche 13 sont connectés l'un à l'autre au niveau de leurs extrémités par un arrondi 17. La coque 11 présente une épaisseur 15 sensiblement constante. Le rebord 12 présente une épaisseur 18 dont une valeur est inférieure à une valeur de l'épaisseur 15 de la coque 11. La portion de coupelle de la coque 11 comporte un sommet 151 sensiblement plat et une hauteur 14. La forme de la coque 11 est choisie de telle sorte à ce qu'elle épouse de manière optimale, lors d'une utilisation sur un pied d'un patient, la forme de la voute plantaire du pied du patient. De ce fait, dans une direction 13, le bord de tranche 13 suit la courbure longitudinale de la voute plantaire, et, dans une direction 16, la coque 11 suit la courbure transversale de la voute plantaire. La forme et les dimensions de la coque 11 sont choisies et adaptées au pied du patient.

La coque 11 est, d'autre part, déformable élastiquement lorsqu'un effort est appliqué au moins sur le sommet 151 de la coque 11. Cette capacité de déformation élastique de la coque 11 permet à l'arche de voute plantaire 10 selon l'invention de suivre tout en contrôlant la déformation naturelle de la voute plantaire d'un patient lorsque ce dernier réalise un pas. A cette fin la coque 11 comporte une âme 112 réaliser dans un premier matériau déformable élastiquement et présentant une souplesse adaptée au patient à traiter. Par exemple, le premier matériau est un matériau plastique, comme un matériau thermoplastique. L'âme 112 de la coque 11 est réalisable par injection du matériau plastique dans un moule de forme adaptée à la forme finale de la coque 11 à réaliser. Dans une variante de réalisation, l'âme 112 de la coque 11 est réalisée dans un matériau présentant des propriétés de mémoire de forme. Il est possible de réaliser l'âme 112 avec un ensemble de matériaux différents de sorte à optimiser la déformation élastique de la coque 11 en fonction des préférences de rééducation à souhaitées.

D'autre part, la coque 11 de l'arche de voute plantaire 10 selon l'invention comporte une couche supérieure 111 qui recouvre le dessus de l'âme 112. La couche supérieure 111 est réalisée en un matériau souple qui peut se déformer de manière élastique. La couche supérieure 111 est fixée sur une surface externe de l'âme 112 de la coque 11. Cette fixation est réalisé par collage, soudure ou surmoulage selon le matériau utilisé pour réalisé la couche supérieure 111. Ainsi, la couche supérieure 111 peut suivre les déformations de l'âme 112 lors d'une utilisation de l'arche de voute plantaire 10 selon l'invention. Le matériau formant la couche supérieure 111 peut être choisi de sorte à pouvoir participer à la réaction de l'arche de voute plantaire 10 selon l'invention lors d'une déformation élastique de cette dernière. D'autre part, la couche supérieure 111 est agencée de sorte à permettre la mise en place et le maintien de l'arche de voute plantaire 10 selon l'invention sur une semelle de sorte à former une semelle orthopédique de rééducation, comme cela sera décrire un peu plus loin dans la description. Le maintien de l'arche de voute plantaire 10 selon l'invention doit être assuré tout laissant la possibilité de retirer pour remplacement ou déplacement l'arche de voute plantaire 10 selon l'invention sans dégradation de cette dernière ou de la semelle. A cette fin, la couche supérieure 111 comporte des moyens de fixation à la semelle réversibles pour que l'arche de voute plantaire 10 selon l'invention soit positionnable de manière amovible, les moyens de fixation formant alors un revêtement de fixation repositionnable. Par exemple, les moyens de fixation réversibles comportent des crochets réalisés sur une surface externe de la couche supérieure 111. Les crochets sont agencés de sorte à pourvoir coopérer, lors d'une mise en place, avec des bouclettes agencées sur une surface de réception de la semelle de l'arche de voute plantaire 10 selon l'invention. Cette surface de réception peut être par exemple recouverte d'un velours à cette fin. Cela forme un système de fixation réversible auto-agrippant qui assure à la fois le maintien de l'arche de voute plantaire 10 selon l'invention sur la semelle et son retrait possible sans dégradation. Un tel système de fixation auto agrippant est connu sous la marque VELCRO^{®}.

De plus, l'arche de voute plantaire 10 selon l'invention comporte aussi une couche inférieure 113 qui recouvre l'intérieur de la coque 11. Comme pour la couche supérieure 111 précédemment décrite, la couche inférieure 113 est réalisée en un matériau souple qui peut se déformer de manière élastique. La couche inférieure 113 est fixée sur une surface interne de l'âme 112 de la coque 11. Cette fixation est réalisé par collage, soudure ou surmoulage selon le matériau utilisé pour réalisé la couche inférieure 113. Ainsi, la couche inférieure 113 peut suivre les déformations de l'âme 112 lors d'une utilisation de l'arche de voute plantaire 10 selon l'invention. Le matériau formant la couche inférieure 113 peut être choisi de sorte à pouvoir compléter la réaction de l'arche de voute plantaire 10 selon l'invention lors d'une déformation élastique de cette dernière. En particulier, le matériau peut être similaire à celui de la surface de la semelle recevant l'arche de voute plantaire 10 selon l'invention, comme par exemple un velours.

La coque 11 est alors composée d'un empilement de couches de matériau souple déformables élastiquement.

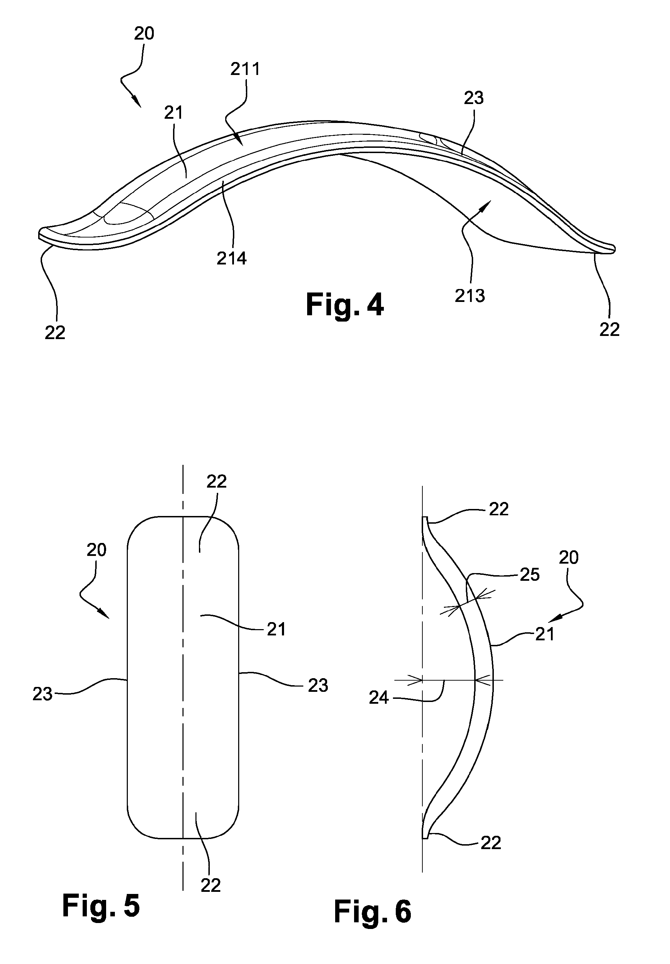

En référence aux figures 4 à 6, nous allons maintenant décrire un deuxième mode de réalisation d'une arche de voute plantaire 20 selon l'invention. L'arche de voute plantaire 20 selon l'invention est structurellement similaire à l'arche de voute plantaire 10 selon l'invention qui vient d'être décrite en détail. L'arche de voute plantaire 20 selon l'invention comporte une coque 21 de forme convexe déformable élastiquement. La coque 21, de manière similaire à la coque 11 précédente, comprend une âme (non représentée) recouverte sur une face supérieure d'une couche supérieure 211 et d'une couche inférieure 213. La coque 21 comporte une portion de cylindre de révolution présentant une flèche 24 et délimité longitudinalement par des rebords 22 et latéralement par des bords de tranche 23. La coque 21 présente une épaisseur 25 qui est réduite au niveau des rebords 22. De nouveau, la forme de la coque 21 est choisie de telle sorte à ce qu'elle épouse de manière optimale, lors d'une utilisation sur un pied d'un patient, la forme de la voute plantaire du pied du patient, dans une direction ou les bords de tranche 23 suivent la courbure longitudinale de la voute plantaire. La forme et les dimensions de la coque 21 sont choisies et adaptées au pied du patient. Les autres caractéristiques de l'arche de voute plantaire 20 selon l'invention sont similaires ou identiques à celles précédemment décrites concernant le premier mode de réalisation de l'arche de voute plantaire 10 selon l'invention.

Quelque soit le mode de réalisation de l'arche de voute plantaire 10,20 selon l'invention qui vient d'être décrit, cette dernière, selon les matériaux choisis pour réaliser la coque 11,21, peut présenter différentes rigidités. Elle est ainsi modulable en fonction de l'évolution de la pathologie et de la rééducation du patient.

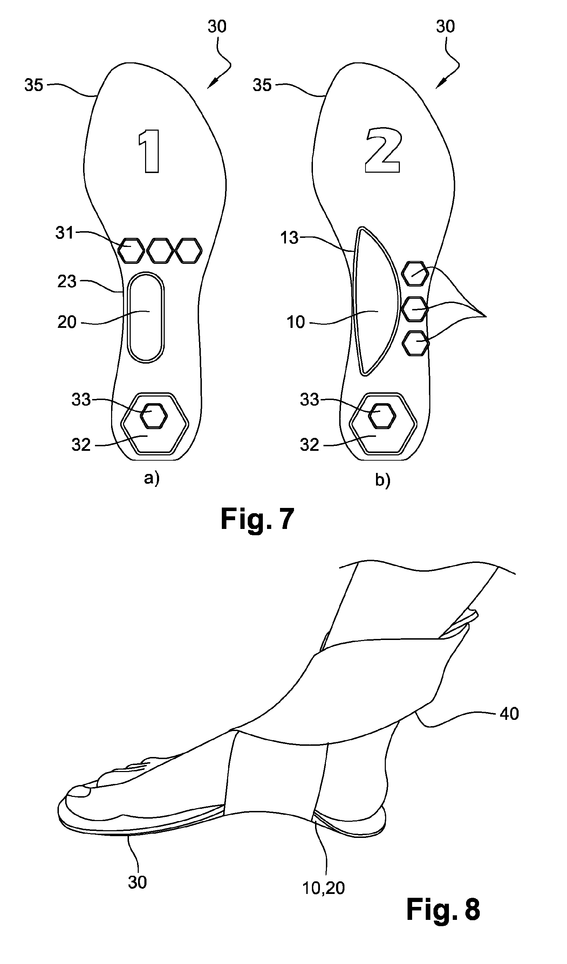

Maintenant, en référence aux figures 7a et 7b, nous allons décrire une semelle orthopédique de rééducation 30 selon l'invention. La semelle orthopédique de rééducation 30 selon l'invention comporte une arche de voute plantaire 10,20 selon l'invention. Cette dernière est positionnée de manière amovible au droit de la voute plantaire d'un pied d'un patient, lors d'une utilisation. La semelle orthopédique de rééducation 30 selon l'invention comporte une semelle 35 dont au moins une partie de la face destinée à recevoir un arche de voute plantaire 10,20 selon l'invention comporte des bouclettes (comme un tissus de velours) pouvant coopérer avec les crochets de la couche supérieure 111,211 de l'arche de voute plantaire 10,20 selon l'invention. La semelle 35 est une semelle fine simple d'épaisseur constante connue ou bien une semelle orthopédique. Afin de compenser les épaisseurs introduites par la mise en place d'une arche de voute plantaire 10,20 selon l'invention, la semelle orthopédique de rééducation 30 selon l'invention comprend une ou plusieurs calles de compensation en mousse 31,32,33 qui sont positionnées le long des rebords 12,22 des l'arche de voute plantaire 10, 20 selon l'invention mise en place. Ces calles participent au confort d'utilisation de la semelle orthopédique de rééducation 30 selon l'invention ainsi réalisée.

Dans ce qui précède, l'arche de voute plantaire 10,20 selon l'invention a été positionnée sur la semelle 35 de sorte à être au droit de l'arche interne de la voute plantaire d'un patient. Toutefois, le pied comporte deux arches supplémentaires qui sont l'arche externe et l'arche antérieure. L'arche de voute plantaire selon l'invention peut être configurée et positionnée au droit de l'arche externe du pied et/ou de l'arche antérieure du pied. Dans une autre variante de réalisation de la semelle orthopédique de rééducation 30, une arche de voute plantaire selon l'invention est configurée et positionnée au droit du talon du pied pour obtenir un effet amortissant.

La semelle orthopédique de rééducation 30 selon l'invention décrite précédemment, est utilisée avec une sangle de maintien 40 avec le pied du patient, la sangle de maintien 40 passant sous la semelle orthopédique de rééducation 30 selon l'invention au droit de l'arche de voute plantaire 10,20 selon l'invention avant de remonter de chaque coté de la cheville du patient et d'être enroulée serrée au dessus de la cheville du patient, formant ainsi un article de marche de pied sous la forme d'une sandale à elles seules, comme cela est illustré en figure 8. La sangle de maintien 40 est amovible. A cette fin, une fixation amovible de la sangle de maintien 40 sur la semelle orthopédique de rééducation 30, et plus particulièrement sur la semelle 35 est réalisée da la même manière que la fixation de l'arche de voute plantaire 10,20 selon l'invention sur ladite semelle 35 : par un système de fixation auto agrippant la sangle de maintien comportant une portion de surface comprenant des crochets destiné à coopérer avec les bouclettes (velours par exemple) de la semelle et/ou de l'arche de voute plantaire 10,20 selon l'invention.

En variante, la sangle de maintien 40 est fixée directement sur la semelle 35 de la semelle orthopédique de rééducation 30 selon l'invention.

L'utilisation d'une telle sangle de maintien 40 avec une arche de voute plantaire selon l'invention permet d'obtenir une action cheville-pied qui soit optimale et novatrice.

Il est à noter que cette sangle maintien 40 peut être utilisée avec une semelle quelconque passive ou plate. La sangle de maintient 40 permet d'avoir une meilleure action correctrice du pied du patient et de sa cheville en même temps. En effet, comme les principaux muscles du pied s'insèrent sur la jambe pour rejoindre le pied, ils passent donc au niveau de la cheville. Ainsi un tel positionnement de la sangle de maintien 40 a un effet important dans la rééducation et/ou la guérison du patient. Elle peut aussi avoir différentes formes et différentes élasticités afin d'être adaptée de manière optimale au patient à traiter.

Dans une autre variante de réalisation, il est possible de fixer sur la couche inférieure de l'arche de voute plantaire selon l'invention des éléments de rigidification de l'arche de voute plantaire selon l'invention, de sorte à bloquer la déformation, lors d'une utilisation, de l'arche de voute plantaire selon l'invention. Bloquer l'élasticité est ainsi possible dans le cas d'une immobilisation obligatoire pour la guérison d'une blessure au pied, et ce pour une durée de six à huit semaines maximum. Après ce laps de temps, il faut absolument enlever les éléments de rigidification pour retrouver une déformation élastique de l'arche de voute plantaire selon l'invention, sans quoi, il y a un risque d'atrophie musculaire. En variante de réalisation ka sangle de maintien 40 peut être positionnée n'importe où sur la semelle orthopédique de rééducation 30.

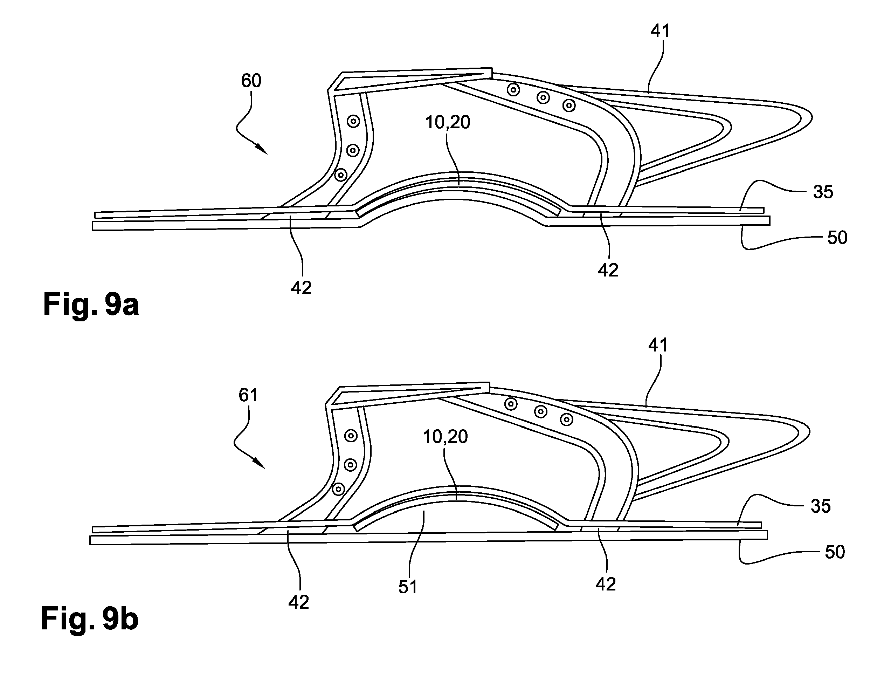

L'ensemble formé de la semelle orthopédique de rééducation 30 selon l'invention et de la sangle de maintien 40 définit un article de marche de pied sous la forme d'une sandale simple. Une semelle extérieure 50, comme illustrée aux figures 9a et 9b, destinée à être en contact avec le sol peut être ajoutée sous la semelle orthopédique de rééducation 30 selon l'invention. La semelle extérieure 50 comporte une surface supérieure recouverte, au moins en partie, avec des crochets pour pouvoir être montée de manière amovible sur le dessous de la semelle orthopédique de rééducation 30 selon l'invention.

Dans un mode de réalisation, la sangle de maintien 40 et l'arche de voute plantaire 10,20 selon l'invention sont prises en sandwich entre la semelle extérieure 50 et la semelle 35. Lors de la mise en place de la semelle extérieure, cette dernière, au niveau de l'arche de voute plantaire selon l'invention, peut soit suivre la courbure de l'arche de voute plantaire selon l'invention en étant fixée sur une surface interne de l'arche de voute plantaire selon l'invention, soit être plane de sorte à réaliser une cavité d'air 51 sous l'arche de voute plantaire selon l'invention.

Dans des variantes de réalisation d'une sandale selon l'invention illustrées aux figures 9a et 9b, la sangle de maintien 40 est remplacée par un système de lanières 41, formant des sangles de maintien différentes, permettant la fixation et le maintien de l'article de marche de pied 60,61 sur le pied et la cheville d'un patient. Le système de lanières 41 est fixé à la semelle 35 et/ou à la semelle extérieure 50 et/ou en sandwich entre les deux semelles au niveau de points de fixation 42. Cette fixation peut être amovible d'une manière similaire à la fixation de la sangle de maintien 40 précédemment décrite.

Il est à noter que les sandales qui viennent d'être décrites présentent la particularité d'être modulables. Toutefois, il est possible de figer une configuration particulière en collant, avec une colle néoprène par exemple, les différents éléments de la sandale que sont la semelle 35, la semelle extérieure 50, la sangle de maintien 40 ou le système de lanières 41, l'arche de voute plantaire 10,20 selon l'invention, les éléments de rigidification. Quelque soit le mode de réalisation, le patient reçoit une correction de la voute plantaire qui est active avec absorption et restitution d'énergie via l'arche de voute plantaire selon l'invention.

En variante d'utilisation, la semelle orthopédique de rééducation 30 selon l'invention qui vient d'être décrite est insérable dans tout article de marche de pied classique comme une chaussure classique d'un patient, l'arche de voute plantaire 10,20 selon l'invention étant alors situé entre la semelle 35 et l'intérieur de la semelle de la chaussure classique.

Il ressort donc de ce qui précède que l'utilisation de l'arche de voute plantaire selon l'invention dans une semelle orthopédique de rééducation 30 permet de réaliser des ensembles avec une voute plantaire élastique suivants :
- Une semelle orthopédique modulable formée par un ou deux types d'arche de voute plantaire amovibles modulables ;
- Une semelle orthopédique-chevillière modulable formée par une arche de voute plantaire amovible associée à une sangle de maintien amovible ;
- Un article de marche comme une sandale orthopédique modulable formée par une arche de voute plantaire, des lanières 41 formant sangle de maintien sur le pied et une semelle externe 50, le tout étant montée de manière amovible ;
- Un article de marche comme une sandale orthopédique figée formée par une arche de voute plantaire, des lanières 41 formant sangle de maintien sur le pied et une semelle externe 50, le tout étant collé par néoprène ou toute autre colle adaptée à cette fin ;
- Un article de marche comme une sandale orthopédique - chevillière modulable formée par une arche de voute plantaire, une sangle de maintien 40, des lanières 41 formant sangle de maintien sur le pied et une semelle externe 50 , le tout étant montée de manière amovible ;
- Un article de marche comme une sandale orthopédique - chevillière figée formée par une arche de voute plantaire, une sangle de maintien 40, des lanières 41 formant sangle de maintien sur le pied et une semelle externe 50 , le tout étant collé par néoprène ou toute autre colle adaptée à cette fin.

Bien entendu, il est possible d'apporter à l'invention de nombreuses modifications sans pour autant sortir du cadre de celle-ci.

## Revendications

1. Arche de voute plantaire destinée à être montée sur une semelle orthopédique de rééducation, au droit de la voute plantaire d'un patient à traiter **caractérisée en ce qu'**elle comporte une coque de forme convexe élastiquement déformable.

2. Arche selon la revendication 1, **caractérisée en ce que** la coque comporte une portion de cylindre de révolution.

3. Arche selon la revendication 1, **caractérisée en ce que** la coque comporte une portion de coupelle, notamment une demi-coupelle.

4. Arche selon l'une des revendications 1 à 3, **caractérisée en ce que** la coque comporte un empilement de couches de matériaux souple.

5. Arche selon la revendication 4, **caractérisée en ce que** la coque comporte une âme, une couche supérieure et une couche inférieure.

6. Arche selon la revendication 5, **caractérisée en ce que** l'âme est réalisée en plastique injecté, notamment en thermoplastique.

7. Arche selon la revendication 5, **caractérisé en ce que** l'âme est réalisée en un matériau à mémoire de forme.

8. Arche selon l'une des revendications 5 à 7, **caractérisée en ce que** la couche supérieure comporte un revêtement de fixation repositionnable, notamment un revêtement de type Velcro®.

9. Semelle orthopédique de rééducation **caractérisée en ce qu'**elle comporte une arche de voute plantaire selon l'une des revendications 1 à 8.

10. Semelle selon la revendication 10, **caractérisée en ce qu'**elle comporte en outre une sangle de maintien de la semelle avec un pied d'un patient.

11. Semelle selon la revendication 11, **caractérisée en ce que** la sangle de maintien est amovible.

12. Semelle selon l'une des revendications 9 à 11, **caractérisé en ce qu'**elle comporte, sous elle, une semelle extérieure, l'arche de voute plantaire étant entre la semelle orthopédique et la semelle extérieure.

13. Semelle selon l'une des revendication 9 à 12, **caractérisé en ce que** l'arche de voute plantaire et/ou la semelle extérieure et/ou la sangle de maintien est (sont) montée(s) de manière amovible(s)

14. Article de marche de pied comportant une semelle, **caractérisée en ce qu'**elle comporte une semelle orthopédique de rééducation selon l'une des revendications 9 à 12.
